# EUROPEAN PATENT APPLICATION

(11) **EP 4 481 051 A1**
(43) Date of publication of application: **25.12.2024**
(21) Application number: 23756460.4
(22) Date of filing: 16.02.2023
(51) Int. Cl.: C12P 19/02, C12N 5/10, C12N 1/15, C12N 1/19, C12N 1/21, C12N 15/61, C12N 15/63, C12N 9/90

(54) **MODIFIED D-ALLULOSE-3-EPIMERASE**

(30) Priority: 18.02.2022 JP 2022023426
(71) Applicant: Amano Enzyme Inc., Nagoya-shi Aichi 460-8630 (JP)
(72) Inventor: YAMASHIRO, Kan, Kakamigahara-shi, Gifu 509-0109 (JP)
(74) Representative: Witthoff Jaekel Steinecke Patentanwälte PartG mbB
(86) International application number: PCT/JP2023/005566
(87) International publication number: WO 2023/157936

(57) **Abstract**

Provided is a heat-resistant D-allulose-3-epimerase.

A polypeptide shown in any one of the following (1) to (3):
(1) a polypeptide consisting of an amino acid sequence having one or two or more amino acid substitutions selected from the group consisting of A56E, N57H, R58G and F111W in the amino acid sequence shown in SEQ ID NO: 1;
(2) a polypeptide consisting of the amino acid sequence having substitution, addition, insertion or deletion of one or several amino acid residues in the amino acid sequence described in the above (1), having D-allulose-3-epimerase activity and retaining one or two or more amino acid substitutions selected from the group consisting of A56E, N57H, R58G, and F111W; and
(3) a polypeptide having a 90% or more sequence identity to the amino acid sequence described in the above (1), having D-allulose-3-epimerase activity and retaining one or two or more amino acid substitutions selected from the group consisting of A56E, N57H, R58G, and F111W.

## Description

### Technical Field

The present invention relates to D-allulose-3-epimerase having acid resistance. More specifically, the present invention relates to modified acid-resistant D-allulose-3-epimerase having improved heat resistance.

### Background Art

Recently, with an increase of health consciousness, the demand for a rare sugar such as allulose having functionality with a low calorie has increased.

Allulose can be synthesized from D-fructose by D-allulose-3-epimerase and an allulose-generating enzyme derived from *Pseudomonas cichorii* or *Arthrobacter globiformis* is known (Patent Literatures 1 and 2).

In producing a rare sugar such as allulose, reactivity in the acidic range is desired to prevent brown discoloration. However, existing enzymes have low reactivity in the acidic range and are not suitably used for treatment in the acidic range. It is known that an allulose-generating enzyme derived from *Dorea* sp. shows relatively high reactivity also in the acidic range (Non Patent Literature 1).

### Citation List

### Patent Literature

Patent Literature 1: JP Patent Publication (Kokai) No. 3-266996A (1991)
Patent Literature 2: WO2014/109254

### Non Patent Literature

Non Patent Literature 1: Zhang 2015 J. Mol. Catal., B Enzym.

### Summary of Invention

### Technical Problem

As described above, an allulose-generating enzyme having relatively high acid resistance is known but its heat resistance was insufficient.

Accordingly, an object of the present invention is to provide D-allulose-3-epimerase having heat resistance.

### Solution to Problem

The present inventor has searched for a microorganism producing allulose-generating enzyme from a strain library of Amano Enzyme Inc. As a result, they found an enzyme having a high reactivity in the acidic range from *Asaia krungthepensis.* They further introduced a specific mutation in a predetermined position of the amino acid sequence of the enzyme. In this manner, they succeeded in obtaining modified D-allulose-3-epimerase having heat resistance.

More specifically, the present invention is as follows.
[1] A polypeptide shown in any one of the following (1) to (3):
   (1) a polypeptide consisting of an amino acid sequence having one or two or more amino acid substitutions selected from the group consisting of A56E, N57H, R58G and F111W in the amino acid sequence shown in SEQ ID NO: 1;
   (2) a polypeptide consisting of an amino acid sequence having substitution, addition, insertion or deletion of one or several amino acid residues in the amino acid sequence described in the above (1), having D-allulose-3-epimerase activity and retaining one or two or more amino acid substitutions selected from the group consisting of A56E, N57H, R58G, and F111W; and
   (3) a polypeptide having a 90% or more sequence identity to the amino acid sequence described in the above (1), having D-allulose-3-epimerase activity and retaining one or two or more amino acid substitutions selected from the group consisting of A56E, N57H, R58G, and F111W.
[2] The polypeptide according to [1], consisting of the amino acid sequence having a single substitution of F111W alone, a triple substitution of A56E, N57H, and R58G or a quadruple substitution of A56E, N57H, R58G and F111W.
[3] The polypeptide according to [1] or [2], having improved heat resistance compared to wild-type D-allulose-3-epimerase consisting of the amino acid sequence represented by SEQ ID NO: 1, in which the polypeptide has 60% or more residual D-allulose-3-epimerase activity when treated at 90°C for 30 minutes.
[4] DNA shown in any one of the following (1) to (3):
   (1) DNA encoding the polypeptide according to [1];
   (2) DNA consisting of a nucleotide sequence represented by any one of SEQ ID NOs: 9 to 14; and
   (3) DNA consisting of an equivalent sequence to the nucleotide sequence represented by any one of SEQ ID NOs: 9 to 14 and encoding a polypeptide having D-allulose-3-epimerase activity.
[5] An expression cassette or recombinant vector including the DNA according to [4].
[6] A transformant obtained by transforming a host with the expression cassette or recombinant vector according to [5].
[7] A method for producing the polypeptide according to any one of [1] to [3], including a step of culturing the transformant according to [6].
[8] A method for producing allulose, including a step of allowing the polypeptide according to any one of [1] to [3] to act on fructose.
[9] Allulose obtained by the production method according to [8].
[10] An enzyme agent including the polypeptide according to any one of [1] to [3].

The specification includes the disclosure of JP Patent Application No. 2022-023426 based on which the priority of the present application claims.

### Advantageous Effects of Invention

According to the present invention, it is possible to obtain a modified D-allulose-3-epimerase having heat resistance. Furthermore, according to the present invention, a method for producing D-allulose and D-allulose produced by the production method are provided.

### Brief Description of Drawings

[Figure 1-1] The figure is a graph showing the optimum pH of the modified D-allulose-3-epimerase of the present invention. Figure 1-1A shows the optimum pH of a wild type and Figure 1-1B shows the optimum pH of mutant 1.
[Figure 1-2] The figure is a graph showing the optimum pH of modified D-allulose-3-epimerase of the present invention. Figure 1-2A shows the optimum pH of mutant 2 and Figure 1-2B shows the optimum pH of mutant 3.
[Figure 2] The figure is a graph showing the optimum temperature of the modified D-allulose-3-epimerase of the present invention.
[Figure 3] The figure is a graph showing the thermal stability of the modified D-allulose-3-epimerase of the present invention.
[Figure 4] The figure is a graph showing the conversion rate to allulose by the modified D-allulose-3-epimerase of the present invention under acidic conditions.

### Description of Embodiments

Now, the present invention will be described in detail.

The present invention is D-allulose-3-epimerase (abbreviation: AkDAE) derived from*A*. *krungthepensis* having at least one amino acid substitution to have improved heat resistance, and a method for producing the enzyme.

Allulose is a monosaccharide having a structure represented by the chemical formula (I) and classified as a ketose. Allulose, which is also called psicose, refers to a rare sugar since it is a naturally-occurring sugar but small in amount.

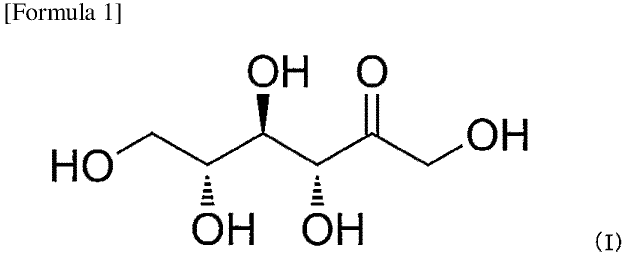

Allulose has sweetness, which is about 70% of that of sucrose, and calorie, which is 1/10 of that of sucrose, and is reported to have functions such as suppression of postprandial glucose levels from increasing and anti-obesity.

### 1. Definition

In the specification, 20 types of amino acid residues in an amino acid sequence are sometimes represented by one-letter codes except in the sequence listing. That is, glycine (Gly) is represented by G, alanine (Ala) A, valine (Val) V, leucine (Leu) L, isoleucine (Ile) I, phenylalanine (Phe) F, tyrosine (Tyr) Y, tryptophan (Trp) W, serin (Ser) S, threonine (Thr) T, cysteine (Cys) C, methionine (Met) M, aspartic acid (Asp) D, glutamic acid (Glu) E, asparagine (Asn) N, glutamine (Gln) Q, lysine (Lys) K, arginine (Arg) R, histidine (His) H, and proline (Pro) P.

In amino acid sequences disclosed in the specification, the left end of them indicates the N terminal and the right end of them indicates the C terminal.

In the specification, examples of "nonpolar amino acids" include alanine, valine, leucine, isoleucine, proline, methionine, phenylalanine, and tryptophan. Examples of "uncharged amino acids" include glycine, serine, threonine, cysteine, tyrosine, asparagine, and glutamine. Examples of "acidic amino acids" include aspartic acid and glutamic acid. Examples of "basic amino acids" include lysine, arginine, and histidine.

In the specification, the "substitution" refers to not only a case where substitution of an amino acid residue is artificially introduced but also a case of a naturally-occurring substitution of an amino acid residue, in other words, a case where displacement with a different amino acid residue already occurred. In the specification, substitution with an amino acid residue may be artificial substitution or naturally-occurring substitution, and artificial substitution is preferable.

The amino acid residue at a mutation site (amino acid residue to be substituted) is expressed by a combination of a one-letter code for an amino acid and the number indicating the position of the amino acid. For example, a case where the 57th arginine is substituted with glycine is expressed as "R57G".

### 2. D-allulose-3-epimerase of the present invention derived from Asaia krungthepensis (A. krungthepensis)

### (i) Polypeptide

D-allulose-3-epimerase of the present invention derived from A. *krungthepensis* is derived from *A*. *krungthepensis* belonging to the genus *Asaia* of the family *Acetobacteraceae* and a type of ketose-3-epimerase.

The amino acid sequence of wild-type D-allulose-3-epimerase derived from *A*. *krungthepensis* is represented by SEQ ID NO: 1. More specifically, the polypeptide consisting of the amino acid sequence represented by SEQ ID NO: 1 is wild-type D-allulose-3-epimerase derived from *A*. *krungthepensis.*

The D-allulose-3-epimerase of the present invention having improved heat resistance consists of any one of the following polypeptides (1) to (3):
(1) a polypeptide consisting of an amino acid sequence having one or two or more amino acid substitutions selected from the group consisting of A56E, N57H, R58G and F111W in the amino acid sequence shown in SEQ ID NO: 1;
(2) a polypeptide consisting of an amino acid sequence having substitution, addition, insertion or deletion of one or several amino acid residues in the amino acid sequence in the amino acid sequence described in the above (1), having D-allulose-3-epimerase activity and retaining one or two or more amino acid substitutions selected from the group consisting of A56E, N57H, R58G and F111W; and
(3) a polypeptide having a 90% or more sequence identity to the amino acid sequence described in the above (1), having D-allulose-3-epimerase activity and retaining one or two or more amino acid substitutions selected from the group consisting of A56E, N57H, R58G, and F111W.

The polypeptides of the above (1) to (3) include not only polypeptides obtained by artificial substitution but also polypeptides obtained by naturally-occurring substitution so as to have such amino acid sequences. The "D-allulose-3-epimerase activity" refers to an activity to generate allulose using fructose as a substrate.

Examples of (1) one or two or more amino acid substitutions selected from the group consisting of A56E, N57H, R58G and F111W include single substitutions such as A56E alone, N57H alone, R58G alone, F111W alone; double substitutions such as A56E/N57H, A56E/R58G, A56E/F111W, N57H/R58G, N57H/F111W, and R58G/F111W; triple substitutions such as A56E/N57H/R58G, A56E/N57H/F111W, A56E/R58G/F111W, and N57H/R58G/F111W and a quadruple substitution such as A56E/N57H/R58G/F111W. The amino acid sequence of a polypeptide having a single substitution of A56E is represented by SEQ ID NO: 3; the amino acid sequence of a polypeptide having a single substitution of N57H is represented by SEQ ID NO: 4; the amino acid sequence of a polypeptide having a single substitution of R58G is represented by SEQ ID NO: 5; the amino acid sequence of a polypeptide having a single substitution of F111W is represented by SEQ ID NO: 6; the amino acid sequence of a polypeptide having a triple substitution of A56E, N57H, and R58G is represented by SEQ ID NO: 7; and the amino acid sequence of a polypeptide having a quadruple substitution of A56E, N57H, R58G and F111W is represented by SEQ ID NO: 8.

In the polypeptide of the above (2), modification of amino acids to be introduced may include a single type of modification selected from substitution, addition, insertion, and deletion (for example, substitution alone) or two types or more modifications (for example, substitution and insertion). In the polypeptide of the above (2), the number of different amino acids at arbitrary different parts may be one or several, for example, 1 to 80, preferably 1 to 70, 1 to 60, 1 to 50, 1 to 40, or 1 to 30, more preferably 1 to 20, 1 to 10, 1 to 9, 1 to 8, 1 to 7, 1 to 6, 1 to 5, or 1 to 4, further preferably 1 to 3, and particularly preferably 1 or 2, or 1. Note that, one or two or more amino acid substitutions selected from the group consisting of A56E, N57H, R58G and F111W of the polypeptide of the above (1) are retained.

In the polypeptide of the above (3), the sequence identity to the amino acid sequence shown in the above (1) may be satisfactorily 70% or more, preferably 80% or more, more preferably 85% or more, further preferably 90% or more, further preferably 95% or more, further preferably 96% or more, further preferably 97% or more, even more preferably 98% or more, and particularly preferably 99% or more.

In the polypeptide of the above (3) herein, the sequence identity to each amino acid sequence shown in the above (1) refers to the sequence identity calculated in comparison with the amino acid sequence shown in the above (1). The "sequence identity" refers to a value of amino acid sequence identity obtained by BLASTPACKAGE [b12seq program (Tatiana A. Tatsusova, Thomas L. Madden, FEMS Microbiol. Lett., Vol. 174, p247-250, 1999) of sgi32 bit edition, Version 2.0.12; available from National Center for Biotechnology Information (NCBI)]. The parameter may be set to Gap insertion Cost value: 11 and Gap extension Cost value: 1.

The polypeptides in the above (2) and (3) are substantially identical to the polypeptide in the above (1).

The D-allulose-3-epimerase of the present invention may be a part of a larger protein (for example, fusion protein). In the fusion protein, examples of the sequence to be added include a sequence useful for purification, such as multiple histidine residues and a sequence to be added ensuring stability at the time of recombinant production.

In the polypeptides in the above (2) and (3), in a case where an amino acid substitution is introduced in the polypeptide of the above (1), a preferable embodiment of the amino acid substitution to be introduced is a conservative substitution. The substitutions in the polypeptides in the above (2) and (3) are, for example, as follows: if the amino acid before substitution is a nonpolar amino acid, substitution with another nonpolar amino acid is made; if the amino acid before substitution is an uncharged amino acid, substitution with another uncharged amino acid is made; if the amino acid before substitution is an acidic amino acid, substitution with another acidic amino acid is made; and if the amino acid before substitution is a basic amino acid, substitution with another basic amino acid is made.

In the polypeptides in the above (2) and (3), "having D-allulose-3-epimerase activity" means that D-allulose-3-epimerase activity can be detected by measurement in accordance with the D-allulose-3-epimerase activation method that will be described later, preferably means the D-allulose-3-epimerase activity is equivalent to that of the polypeptide of the above (1), and more specifically means that relative activity to the D-allulose-3-epimerase activity of the polypeptide of the above (1) regarded as 1, is about 0.8 to 1.2.

The polypeptides of the above (1) to (3) are called D-allulose-3-epimerase derived from *A*. *krungthepensis* as long as they have D-allulose-3-epimerase activity.

### (ii) DNA encoding the enzyme

The DNA encoding the D-allulose-3-epimerase of the present invention is any one of the DNAs shown in the following (a) to (c):
(a) DNA encoding the polypeptide shown in any one of the following (1) to (3):
   (1) a polypeptide consisting of an amino acid sequence having one or two or more amino acid substitutions selected from the group consisting of A56E, N57H, R58G and F111W in the amino acid sequence shown in SEQ ID NO:1;
   (2) a polypeptide consisting of the amino acid sequence having substitution, addition, insertion or deletion of one or several amino acid residues in the amino acid sequence described in the above (1), having D-allulose-3-epimerase activity and retaining one or two or more amino acid substitutions selected from the group consisting of A56E, N57H, R58G, and F111W; and
   (3) a polypeptide having a 90% or more sequence identity to the amino acid sequence described in the above (1), having D-allulose-3-epimerase activity and retaining one or two or more amino acid substitutions selected from the group consisting of A56E, N57H, R58G, and F111W;
(b) DNA consisting of a nucleotide sequence represented by any one of SEQ ID NOs: 9 to 14; and
(c) DNA containing an equivalent sequence to the nucleotide sequence of DNA of the above (a) or (b) and encoding a polypeptide having D-allulose-3-epimerase activity.

Examples of the nucleotide sequence of the DNA encoding the amino acid sequence represented by SEQ ID NO: 1 include nucleotide sequences represented by SEQ ID NO: 2 and SEQ ID NO: 15. The nucleotide sequence represented by SEQ ID NO: 15 is the nucleotide sequence of DNA encoding wild type D-allulose-3-epimerase derived from *A*. *krungthepensis,* and the nucleotide sequence represented by SEQ ID NO: 2 is the nucleotide sequence obtained by changing the nucleotide sequence represented by SEQ ID NO: 15 so as to have codons suitable for *Escherichia coli.* Thus, the DNA of the present invention can be appropriately designed by those skilled in the art using the nucleotide sequence represented by SEQ ID NO: 2 as a standard sequence.

Examples of the DNA encoding the D-allulose-3-epimerase of the present invention include DNAs consisting of the nucleotide sequences represented by SEQ ID NOs: 9 to 14. The nucleotide sequence represented by SEQ ID NO: 9 is a DNA sequence encoding a polypeptide consisting of the amino acid sequence of an A56E mutant represented by SEQ ID NO: 3; the nucleotide sequence represented by SEQ ID NO: 10 is a DNA sequence encoding a polypeptide consisting of the amino acid sequence of an N57H mutant represented by SEQ ID NO: 4; the nucleotide sequence represented by SEQ ID NO: 11 is a DNA sequence encoding a polypeptide consisting of the amino acid sequence of an R58G mutant represented by SEQ ID NO: 5; the nucleotide sequence represented by SEQ ID NO: 12 is a DNA sequence encoding a polypeptide consisting of the amino acid sequence of an F111W mutant represented by SEQ ID NO: 6; the nucleotide sequence represented by SEQ ID NO: 13 is a DNA sequence encoding a polypeptide consisting of the amino acid sequence of an A56E/N57H/R58G triple mutant represented by SEQ ID NO: 7; and the nucleotide sequence represented by SEQ ID NO: 14 is a DNA sequence encoding a polypeptide consisting of the amino acid sequence of an A56E/N57H/R58G/F111W quadruple mutant represented by SEQ ID NO: 8.

Note that, the nucleotide sequences represented by SEQ ID NOs: 9 to 14 are the nucleotide sequences obtained by changing so as to have codons suitable for *Escherichia coli.*

The DNA encoding the D-allulose-3-epimerase of the present invention is not limited to the above sequences and may be DNAs having equivalent sequences to the above sequences. The equivalent sequence herein refers to a nucleotide sequence having a homology of 70% or more, preferably 80% or more, more preferably 85% or more, further preferably 90% or more, further preferably 95% or more, further preferably 96% or more, further preferably 97% or more, even more preferably 98% or more, and particularly preferably 99% or more to the nucleotide sequence of DNA shown in (1) or (2). DNA having such a nucleotide sequence is included in the DNA encoding the D-allulose-3-epimerase of the present invention as long as it encodes a polypeptide having D-allulose-3-epimerase activity.

The "homology" of DNA is calculated using software open-to-public or commercially available software having algorithm using a standard sequence as a reference sequence. More specifically, e.g., BLAST, FASTA, or GENETYX (manufactured by SOFTWARE KNOWLEDGE INCORPORATED) can be used. These may be used by default parameters.

The nucleotide sequence having substitution, addition, insertion or deletion of several nucleotides corresponding the aforementioned substitution, addition, insertion or deletion in an amino acid sequence is included in the DNA encoding the D-allulose-3-epimerase of the present invention as long as it encodes a polypeptide having D-allulose-3-epimerase activity.

Furthermore, a nucleotide sequence complementary to DNA consisting of a nucleotide sequence encoding the aforementioned polypeptide is an equivalent sequence, and DNA hybridizing with DNA consisting of the nucleotide sequence mentioned above under a stringent condition is included in the DNA encoding the D-allulose-3-epimerase of the present invention as long as it encodes a polypeptide having D-allulose-3-epimerase activity.

Examples of the stringent condition herein include, for example, a condition for hybridization performed by placing nylon membrane having DNA immobilized thereon together with a probe in a 6 × SSC solution (1 × SSC solution is prepared by dissolving sodium chloride (8.76 g) and sodium citrate (4.41 g) in 1 liter of water), 1% SDS, 100 µg/mL salmon sperm DNA, 0.1% bovine serum albumin, 0.1% polyvinylpyrrolidone, and 0.1% Ficoll (in the specification, "%" refers to "w/v") while keeping them at 65°C for 20 hours. However, the stringent condition is not limited to this. The hybridization conditions can be set by those skilled in the art in consideration of other conditions such as a probe concentration, the length of a probe and reaction time in addition to the salt concentration and temperature and the like of such a buffer solution.

The specific procedure of the hybridization method may refer to, e.g., Molecular Cloning, A Laboratory Manual 2nd ed. (Cold Spring Harbor Laboratory Press (1989)).

Now, a method for obtaining the DNA encoding the D-allulose-3-epimerase of the present invention by hybridization will be described below, but the method for obtaining the DNA encoding the D-allulose-3-epimerase of the present invention is not limited to the following method.

First, DNA obtained from an appropriate gene source is connected to a plasmid or a phage vector in accordance with a routine method to prepare a DNA library. The library is introduced to an appropriate host. The transformants obtained are cultured on a plate. Grown colonies or plaque are transferred to a nitrocellulose or nylon membrane and denatured. In this manner, DNAs are immobilized on the film. The film is placed in a solution having the aforementioned composition and containing a probe labeled with, e.g., ³²P and kept warm in the above stringent conditions to perform hybridization. As the probe, a polynucleotide encoding all or part of the amino acid sequence represented by SEQ ID NO: 1 or a polynucleotide encoding all or part of the nucleotide sequence represented by SEQ ID NO: 2 can be used.

After completion of the hybridization, a probe non-specifically adsorbed is washed away. A clone hybridized with the probe is identified by e.g., autoradiography. This operation is repeated until the hybrid-formation clone can be isolated. Finally, a gene encoding a protein having a desired enzyme activity is selected from the obtained clones. The isolation of the gene can be carried out by a commonly known polynucleotide extraction method such as an alkaline method.

The DNA encoding the D-allulose-3-epimerase of the present invention can be isolated from a microorganism producing the predetermined polypeptide. For example, a desired DNA can be isolated from the genome of the microorganism by PCR or a hybridization method using the genomic DNA of *Asaia krungthepensis* as a template and a primer or probe designed in consideration of degeneracy of a gene based on known amino acid sequence information or a primer or probe designed based on known nucleotide sequence information.

The DNA encoding the D-allulose-3-epimerase of the present invention includes various types of DNAs due to codon degeneracy. Various types of DNAs encoding the same amino acid sequence are likely to be artificially prepared by use of commonly known genetic engineering methods. For example, in producing a protein by genetical engineering, when a codon used on an original gene encoding a desired protein has a low use frequency in a host, the expression level of the protein is sometimes low. In this case, the expression of the desired protein can be enhanced by optimizing a codon usage frequency to a host without changing the amino acid sequence encoded.

As an index representing the codon usage frequency, the total host optimal codon usage frequency of each codon may be employed. The optimum codon is defined as the most frequently used one of the codons corresponding to the same amino acid. The frequency of codon usage is not particularly limited as long as it is optimized for a host, for example, examples of optimal codons in *Escherichia coli* are as follows. F: phenylalanine (ttt), L: leucine (ctg), I: isoleucine (att), M: methionine (atg), V: valine (gtg), Y: tyrosine (tat), stop codon (taa), H: histidine (cat), Q: glutamine (cag), N: asparagine (aat), K: lysine (aaa), D: aspartic acid (gat), E: glutamic acid (gaa), S: serin (agc), P: proline (ccg), T: threonine (acc), A: alanine (gcg), C: cysteine (tgc), W: tryptophan (tgg), R: arginine (cgc), and G: glycine (ggc).

Examples of a method that can be used for artificially modifying an amino acid sequence by introducing a mutation into a gene include commonly known methods such as the Kunkel method and the Gapped duplex method, and a mutation introduction kit using site-directed mutagenesis such as QuikChange^{™} Site-Directed Mutagenesis Kit (Stratagene), GeneArt^{™} Site-Directed Mutagenesis PLUS System (Invitrogen), TaKaRa Site-Directed Mutagenesis System (Mutan-K, Mutan-Super Express Km, PrimeSTAR Mutagenesis Basal Kit, etc.: Takara Bio Inc.).

The nucleotide sequence of DNA can be confirmed by sequencing according to a routine method, such as the dideoxynucleotide chain termination method (Sanger et al. (1977), Proc. Natl. Acad. Sci. USA 74: 5463). Also, the sequence can be analyzed using an appropriate DNA sequencer.

Whether or not the DNA obtained is DNA encoding a desired polypeptide is confirmed by comparing the sequenced nucleotide sequence with the nucleotide sequence represented by SEQ ID NO: 2, or by comparing the amino acid sequence estimated from the determined nucleotide sequence with the amino acid sequence represented by SEQ ID NO: 1.

### (iii) Expression cassette or recombinant vector

An expression cassette or recombinant vector containing DNA encoding the predetermined polypeptide (hereinafter also referred to as the "expression cassette of the present invention" or "recombinant vector of the present invention") can be obtained by linking a promoter and a terminator to the DNA encoding the D-allulose-3-epimerase of the present invention or inserting the expression cassette of the present invention or the DNA encoding the D-allulose-3-epimerase of the present invention into an expression vector.

The expression cassette of the present invention or the recombinant vector of the present invention may contain, if necessary, transcription elements such as an enhancer, CCAAT box, TATA box and SPI site, as control factors, other than a promoter and a terminator. It is sufficient that these control factors are operatively linked to the DNA of the present invention. The operatively linking refers to linking of various control factors regulating the DNA of the present invention to the DNA of the present invention such that they can be operated in a host cell.

The recombinant vector of the present invention serving as an expression vector is preferably constructed for genetic recombination from a phage, plasmid, or virus that can autonomously proliferate within a host. Such an expression vector is commonly known. Examples of commercially available expression vectors include pQE vector (QIAGEN K.K.), pDR540, pRIT2T (GE Healthcare Bioscience), and pET vector (Merck). The expression vector can be selected in consideration of the combination appropriate for a host cell, and put in use. For example, in a case where *Escherichia coli* is a host cell, a combination of pET vector and DH5α E. coli strain, a combination of pET vector and BL21 (DE3) *E. coli* strain, a combination of pDR540 vector and JM109 *E. coli* strain, or the like, is mentioned.

### (iv) Transformant

A transformant (hereinafter referred to as "the transformant of the present invention") is obtained by transforming a host with the expression cassette or recombinant vector of the present invention.

The host for use in producing a transformant is not particularly limited as long as a gene can be introduced into it, an expression cassette or recombinant vector is stable therein, and it can autonomously reproduce and express traits of a gene containing the DNA of the present invention. Examples of the host suitably used include, bacteria belonging to the genus *Escherichia* such as *Escherichia coli,* the genus *Bacillus* such as *Bacillus subtilis,* the genus *Pseudomonas* such as *Pseudomonas putida,* the genus *Asaia* such as *Asaia krungthepensis,* and yeasts. Other than these, e.g., animal cells, insect cells and plant may be used.

The transformant of the present invention can be obtained by introducing the expression cassette of the present invention or the recombinant vector of the present invention into a host. The site into which the DNA of the present invention is introduced is not particularly limited as long as a desired gene can be expressed and may be on a plasmid or on the genome. As the method for introducing the expression cassette of the present invention or the recombinant vector of the present invention, a recombinant vector method and a genome editing method can be specifically mentioned. Conditions for introducing an expression cassette or a recombinant vector into a host may be appropriately set depending on, e.g., the type of host. If the host is a bacterium, for example, a method of using a competent cell obtained by calcium ion treatment and an electroporation method may be mentioned. If the host is a yeast, for example, an electroporation method, a spheroplast method and a lithium acetate method may be mentioned. If the host is an animal cell, for example, an electroporation method, a calcium phosphate method and a lipofection method may be mentioned. If the host is an insect cell, for example, a calcium phosphate method, a lipofection method and an electroporation method may be mentioned. If the host is a plant vacuole, for example, an electroporation method, an agrobacterium method, a particle gun method, and a PEG method may be mentioned.

Whether or not the expression cassette of the present invention or the recombinant vector of the present invention is incorporated in a host can be checked by a method such as a PCR method, Southern hybridization, and Northern hybridization

In the case of checking by PCR method whether or not the expression cassette of the present invention or the recombinant vector of the present invention is incorporated into a host, it is only necessary to separate, for example, genomic DNA, an expression cassette or a recombinant vector from a transformant and purify it.

The separation and purification of an expression cassette or recombinant vector are performed, for example, in case where the host is a bacterium, for a lysate obtained by bacteriolysis. The bacteriolysis is performed by applying a treatment with a lytic enzyme such as lysozyme, if necessary, in combination with a protease, and other enzymes, and a surfactant such as sodium lauryl sulfate (SDS).

In addition, a physical method for crushing cells by freeze-thaw treatment and processing by a French press may be used in combination. DNA is separated and purified from a lysate by, for example, appropriately combining a protein removal treatment with phenol and a protease, a treatment with ribonuclease, precipitation with alcohol, and a commercially available kit.

DNA can be cleaved by a routine method such as a treatment with a restriction enzyme. As the restriction enzyme, for example, type II restriction enzyme that acts on a predetermined nucleotide sequence is used. DNA is ligated to an expression cassette or an expression vector, for example, by use of DNA ligase.

Thereafter, a primer specific to the DNA of the present invention is designed and PCR is performed using the DNA separated and purified as a template. The amplification product obtained by PCR is subjected to, e.g., agarose gel electrophoresis, polyacrylamide gel electrophoresis or capillary electrophoresis and staining with, e.g., ethidium bromide and SYBR Green solution. The amplification product can be detected as a band. In this manner, it can be confirmed that the host was transformed.

Alternatively, PCR can also be performed using a primer previously labeled with, e.g., a fluorescent dye to detect an amplification product. As another method which may be employed, the amplification product is bound to a solid phase such as a microplate and confirmed by, e.g., a fluorescence and enzymatic reaction.

### 3. Enzymological properties

The polypeptide of the present invention, i.e., D-allulose-3-epimerase has the following enzymological properties:

### (i) Action

The polypeptide of the present invention generates allulose from fructose used as a substrate. For example, in a case where 0.1 U/mL polypeptide is used for treatment and 30 to 60 (w/v)% fructose is used as a substrate at 75°C for 24 hours, the conversion rate of fructose to allulose at pH5.3 is 15% or more, preferably 20% or more, further preferably 25% or more, and particularly preferably 27% or more.

### (ii) Optimum pH

The optimum pH of the polypeptide of the present invention is 7.5. The activity thereof within the acidic range of pH5.0 to 7.0 is 40% or more of the activity at pH7.5, preferably 50% or more, and more preferably 60% or more. The optimum pH is a value obtained by measurement in a 50 mM acetate buffer (pH4.0 to 5.0) and a 50 mM phosphate buffer (pH5.0 to 7.5). The optimum pH of wild-type D-allulose-3-epimerase measured in the same conditions is equivalent.

### (iii) Optimum temperature

The optimum temperature of the polypeptide of the present invention in the range of 50 to 90°C is preferably 90°C. The activity thereof at 80 to 90°C is 70% or more of the activity measured at the optimum temperature. The optimum temperature is a value obtained by measurement using a 50 mM Tris-HCl buffer at pH7.5. The optimum temperature of wild-type D-allulose-3-epimerase measured in the same condition is about 70°C.

### (iv) Thermal stability

The polypeptide of the present invention is stable up to 70°C, preferably up to 80°C, more preferably 85°C and even more preferably up to 90°C. When the peptide is treated in each of the temperature conditions for 30 minutes, the residual activity at 80°C is 60% or more, preferably 90% or more, and further preferably about 100%; the residual activity at 85°C is 30% or more, preferably 90% or more, further preferably about 100%; and the residual activity at 90°C is preferably 60% or more, further preferably 90% or more, and further preferably about 100%. The residual activity of wild-type D-allulose-3-epimerase measured in the same condition up to about 70°C is equivalent to the case of the polypeptide of the present invention but the residual activity of the case treated at 80°C for 30 minutes is 50% or less, and the residual activity of the case treated at 85°C for 30 minutes is almost 0. In short, the polypeptide of the present invention has higher thermal stability than wild-type D-allulose-3-epimerase.

### (v) Substrate specificity

When D-fructose is used as a substrate, D-allulose-3-epimerase generates D-allulose. When D-tagatose is used as a substrate, D-allulose-3-epimerase generates D-sorbose. The epimerase activity of D-allulose-3-epimerase is a reversible reaction. Accordingly, when D-allulose is used as a substrate, D-allulose-3-epimerase generates D-fructose, whereas when D-sorbose is used as a substrate, D-allulose-3-epimerase generates D-tagatose. The substrate specificity (reactivity) to D-allulose is the highest.

### 4. Method for producing enzyme

The present invention includes a method for producing a polypeptide, including a step of culturing a transformant as mentioned above.

Culture conditions for the step of culturing a transformant may be appropriately set in consideration of the nutritional physiological properties of the above transformant but liquid culture is preferable. If production is performed industrially, stirred culture with aeration is preferably employed. As a nutritional source for a culture medium, a nutritional source required for the growth of a transformant is used. As a carbon source, any carbon compound may be used as long as it can be utilized. Examples of carbon compounds include glucose, sucrose, lactose, maltose, molasses and pyruvic acid. As a nitrogen source, any nitrogen compound may be used as long as it can be utilized. Examples of nitrogen compounds include peptone, meat extract, yeast extract, casein hydrolysate and a soybean meal alkaline extract. Other than the carbon source and nitrogen source, for example, a phosphate, a carbonate, a sulfate, salts of magnesium, calcium, potassium, iron, manganese and zinc, predetermined amino acids and predetermined vitamins may be used, if necessary.

The culture temperature may be appropriately set within the range for the above transformant to grow and for the above transformant to produce a predetermined polypeptide. The culture temperature is preferably, e.g., about 15 to 37°C. Culture may be terminated at an appropriate time when the yield of a polypeptide reaches the highest. The culture time is usually about 12 to 48 hours.

After the step of culturing a transformant, the culture solution is subjected to e.g., centrifugation to collect the culture supernatant and/or bacterial cells. The bacterial cells are lysed by applying a mechanical means such as ultrasonic wave and French press or a lytic enzyme such as lysozyme to them, and, if necessary, using an enzyme such as a protease or a surfactant such as sodium lauryl sulfate (SDS) to obtain a water-soluble fraction containing a predetermined oxygenase. If an appropriate expression cassette or expression vector and a host are selected, the expressed polypeptide can be secreted in a culture medium.

The water-soluble fraction containing a polypeptide obtained as described above may be directly subjected to purification treatment. Alternatively, the polypeptide in the water-soluble fraction may be concentrated and then subjected to purification treatment. The concentration can be performed by, for example, vacuum concentration, membrane concentration, salting out treatment or fractional precipitation with a hydrophilic organic solvent (for example, methanol, ethanol and acetone). The purification treatment can be performed by appropriately combining, for example, gel filtration, adsorption chromatography, ion exchange chromatography and affinity chromatography. The predetermined polypeptide purified may be pulverized, if necessary, by, e.g., lyophilization, vacuum dry, or spray dry.

Further, a sugar chain and/or a lipid may be added or modification can be made such that processing of the N-terminal or C-terminal occurs. The modification as mentioned above enables, e.g., extraction of a recombinant protein, simplified purification or addition of a biological function.

Note that, if *A*. *krungthepensis* is mutated to produce D-allulose-3-epimerase according to any one of (1) to (3) in the above 2. (i), the *A*. *krungthepensis* mutant is cultured and the D-allulose-3-epimerase of the present invention is collected from the culture solution or bacterial cells, and purified to produce D-allulose-3-epimerase.

### 5. Enzyme agent

The D-allulose-3-epimerase of the present invention can be provided as an enzyme agent. The enzyme agent of the present invention contains the enzyme of the present invention as an active ingredient. Also, the enzyme agent of the present invention can be provided in the form of microbial cells containing the enzyme of the present invention within the cells. The enzyme agent includes microbial cells containing a gene encoding the enzyme of the present invention within the cells. The microorganism is not particularly limited as long as it expresses the enzyme of the present invention and accumulates it within the bacterial cells. Examples of the microorganism include *Escherichia coli, Bacillus* genus bacteria, acetic acid bacteria, filamentous fungi, actinomycetes and yeasts. The microorganism is preferably *Escherichia coli.* The microbial cells can be prepared as transformants by introducing the gene of the enzyme into a microorganism. The transformant can be prepared by a commonly known method. The transformant may be brought into contact with fructose to act on fructose. The phrase "to act on fructose" means that a transformant is brought into contact with fructose to induce an enzymatic reaction. The transformant expresses an enzyme within a cell and the enzyme acts on fructose. After the enzyme acts on fructose and fructose produces allulose, the microorganism may be destroyed by, e.g., ultrasonic wave. The cell debris may be removed by, e.g., centrifugation. The enzyme agent of the present invention may contain an excipient, a buffering agent, a suspending agent, a stabilizer, a preservative, an antiseptic agent and saline, other than an active ingredient (the enzyme of the present invention). Examples of the excipient that can be used include starch, dextrin, maltose, trehalose, lactose, D-glucose, sorbitol, D-mannitol, white sugar and glycerol. Examples of the buffering agent that can be used include a phosphate, a citrate and an acetate. Examples of the stabilizer that can be used include propylene glycol and ascorbic acid and a heavy metal such as manganese, cobalt, magnesium or salts of these. Examples of the preservative that can be used include phenol, benzalkonium chloride, benzyl alcohol, chlorobutanol, and methylparaben. Examples of the antiseptic agent that can be used include ethanol, benzalkonium chloride, paraoxybenzoic acid and chlorobutanol. The content of the active ingredient (the enzyme of the present invention) of the enzyme agent is not particularly limited as long as a desired ketose can be generated. The enzyme agent of the present invention can be provided usually in a solid state (for example, an immobilized enzyme having the enzyme immobilized onto the surface or interior of a material to which the enzyme can be fixed, such as a granule, a powder, silica or a porous polymer), or in a liquid state.

### 6. Usage of enzyme

Allulose can be generated using the D-allulose-3-epimerase of the present invention, and using fructose as a substrate. Another type of ketose can be generated by changing a substrate. For example, D-sorbose can be generated using D-tagatose as a substrate. D-tagatose can be generated using D-sorbose as a substrate. The optimum pH of D-allulose-3-epimerase of the present invention is low and the heat resistance thereof is high. Because of this, allulose can be generated in a low pH condition and a high-temperature condition. A reducing sugar containing allulose is likely to change into brown and brown discoloration can be prevented in the acidic region. Therefore, to prevent brown discoloration, generation may be made preferably at low pH. In the high-temperature condition, since the solubility of a substrate increases, the substrate can be used in a high concentration, with the result that a highly concentrated allulose can be generated. The temperature condition of the enzymatic reaction is 30 to 95°C, preferably 40 to 90°C, and more preferably 45 to 80°C. The pH condition is pH 4.0 or more and less than pH 8.0, preferably pH 4.0 or more and less than pH 7.5, further preferably pH 4.0 or more and less than pH 7.0, or pH 4.0 or pH 4.5 or more and pH 6.5, pH 6.0, pH 5.5 or less.

When the enzymatic reaction is performed, it is preferable that the substrate concentration is 30 to 60 (w/v)%, the reaction time is preferably 10 minutes to 48 hours and more preferably 10 minutes to 24 hours, 10 minutes to 12 hours, 10 minutes to 6 hours, 10 minutes to 3 hours, 10 minutes to 2 hours and 10 minutes to 1 hour.

When a reaction is performed by using 30 to 60 (w/v)% fructose as a substrate, for example, in the case where a 0.1 to 0.3 U/mL enzyme sample (0.01 mL) is added to 1 mL of a substrate solution (45.0 (w/v)% fructose, 100 mM acetate buffer, 1 mM cobalt chloride, pH 5.3) and the mixture is treated at 75°C for 24 hours, the conversion rate to allulose is 15% or more, preferably 20% or more, and further preferably 25% or more.

### Examples

The present invention will be more specifically described by the following Examples but the present invention is not limited by these Examples.

### 1. Acquisition and identification of enzyme

A mutation was introduced by performing PCR using a full-length sequence of D-allulose-3-epimerase (SEQ ID NO: 2) introduced in pET24 vector as a template, the following primers and PrimeSTAR Mutagenesis Basal Kit (Takara Bio Inc.) in accordance with a routine method.

### (Primer for mutagenesis at positions 56 to 58)

Rv: 5'-GAAGAACATGGCCTGGGCATTACCATGAGC-3' (SEQ ID NO: 16)
Fw: 5'-CAGGCCATGTTCTTCCAGTTCGCGCAGGGT-3' (SEQ ID NO: 17) (primer for mutagenesis at position 111)
Rv: 5'-AGCGCGTGGCAGAAATATGCGACCCCG-3' (SEQ ID NO: 18)
Fw: 5'-TTTCTGCCACGCGCTATACAGAATGCC-3' (SEQ ID NO: 19)

*E. coli* JM109 was transformed by the obtained amplification product to obtain a gene expression vector. The presence of D-allulose-3-epimerase sequence introduced in the gene expression vector was confirmed. The gene expression vector was introduced into *E. coli* BL21 (DE3). The transformant was cultured with shaking in LB medium at 37°C for 3 hours. Thereafter, shaking culture was performed at 25°C for 20 minutes and then IPTG (final concentration: 1 mM) was added and shaking culture was performed at 25°C for 24 hours. Bacterial cells were collected from the culture solution, suspended in a 50 mM Tris-HCl buffer (pH 7.5), crushed by a beads shocker and centrifuged at 15,000 rpm for 10 minutes. The supernatant after centrifugation was recovered and used as an enzyme sample.

In the following Examples, the enzyme samples containing polypeptides having the following amino acid sequences as D-allulose-3-epimerase were used.

Wild type: SEQ ID NO: 1
Mutant 1: SEQ ID NO: 7 (A56E, N57H, R58G)
Mutant 2: SEQ ID NO: 6 (F111W)
Mutant 3: SEQ ID NO: 8 (A56E, N57H, R58 G, F111W)

### (1) Method for measuring activity

### (D-allulose-3-epimerase activity)

To 1 mL of a substrate solution (45.0 (w/v)% fructose, 50 mM Tris-HCl buffer, 1 mM cobalt chloride, pH 7.5), the enzyme sample (0.01 mL) diluted in an appropriate concentration was added. The mixture was treated at 50°C for one hour and thereafter subjected to a boiling treatment for 10 minutes. After the sample was allowed to cool, fructose and allulose were detected by HPLC. Provided that the amount of the enzyme generating 1 µmol of allulose per minute was defined as 1 unit, the activity of D-allulose-3-epimerase was obtained.

### (2) Enzymological property evaluation test

### Optimum pH

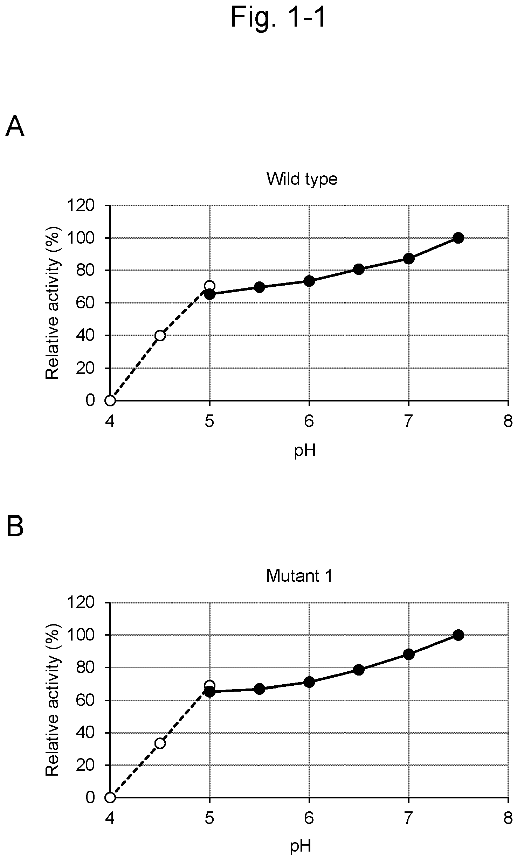

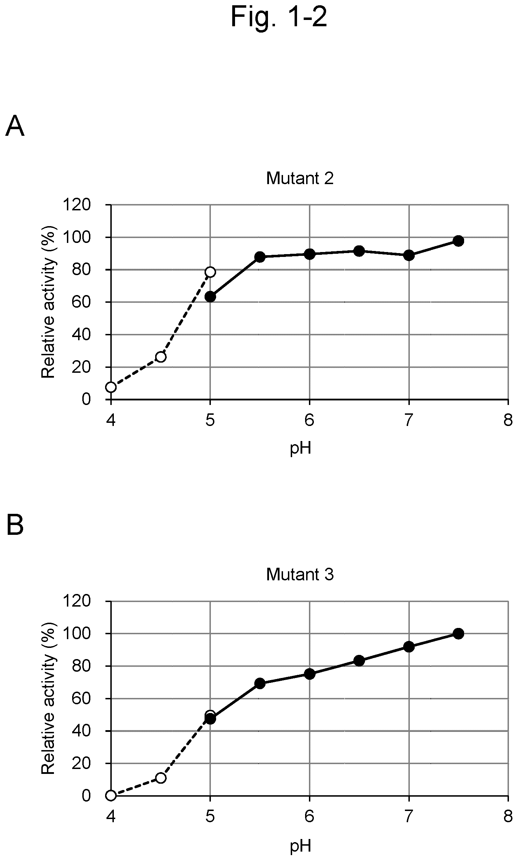

Optimum pH in the range of pH 4.0 to 7.5 was checked. D-allulose-3-epimerase activity was determined using a solution containing 45.0 (w/v)% fructose, 50 mM acetate buffer (pH 4.0 to 5.0) or a 50 mM phosphate buffer (pH 5.0 to 7.5) and 1 mM cobalt chloride as a substrate solution. The activity was evaluated as relative activity to the activity at pH 7.5 as 100%. The results are shown in Figures 1-1 and 1-2. As shown in Figures 1-1 and 1-2, the activity of any one of the D-allulose-3-epimerases was the highest at pH 7.5. The relative activities of D-allulose-3-epimerases of the wild type (SEQ ID NO: 1), mutant 1 (SEQ ID NO: 7) and mutant 2 (SEQ ID NO: 6) at pH 5.0 to 7.0 to the activity at pH 7.5 were 60% or more. The relative activity of D-allulose-3-epimerase of mutant 3 (SEQ ID NO: 8) in the range of pH 5.0 to 7.0 to the activity at pH 7.5 was 40% or more at pH5.0 and 60% or more at pH 5.5 to 7.0.

### Optimum temperature

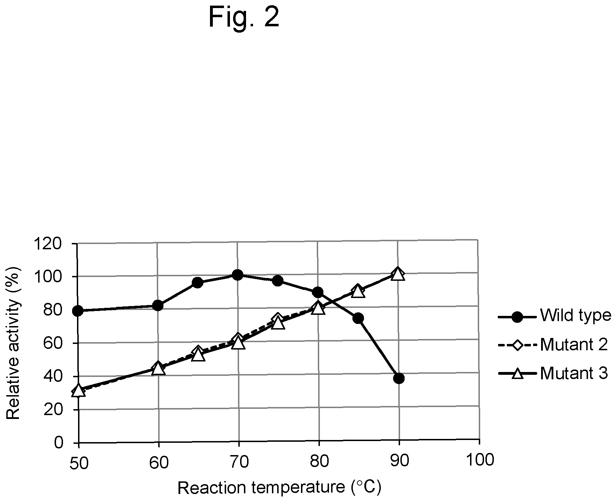

The optimum temperature in the range of 50 to 90°C was checked. The D-allulose-3-epimerase activity was determined using a substrate solution containing 45.0 (w/v)% fructose, a 50 mM Tris-HCl buffer (pH 7.5) and 1 mM cobalt chloride, while changing the reaction temperature to 50°C, 60°C, 65°C, 70°C, 75°C, 80°C, 85°C and 90°C. The activities at individual temperatures were evaluated relative to the highest activity as 100%. The results are shown in Figure 2. As shown in Figure 2, wild-type (SEQ ID NO: 1) D-allulose-3-epimerase exhibited the highest activity at 70°C. In contrast, D-allulose-3-epimerases of mutant 2 (SEQ ID NO: 6) and mutant 3 (SEQ ID NO: 8) exhibited the highest activity at 90°C.

### Thermal stability

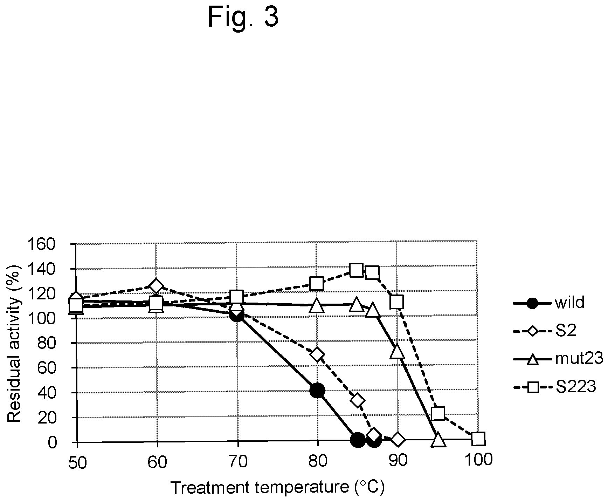

Thermal stability was checked. Enzyme samples diluted so as to have 50 U/mL were pretreated at 50 to 100°C for 30 minutes. After the samples were allowed to cool, the activity of D-allulose-3-epimerase was checked. The relative activities to the activity of an untreated sample as 100% were evaluated. The results are shown in Figure 3. As shown in Figure 3, the residual activity of D-allulose-3-epimerase of any one of the samples was stable up to 70°C, and exhibited 100% by a treatment for 30 minutes. The residual activity of D-allulose-3-epimerase of mutant 1 (SEQ ID NO: 7) was 60% or more at 80°C and higher than the residual activity (40% or less) of D-allulose-3-epimerase of wild-type (SEQ ID NO: 1). The residual activity of D-allulose-3-epimerase of mutant 2 (SEQ ID NO: 6) was stable up to 85°C and exhibited 100% by a treatment for 30 minutes. The residual activity of D-allulose-3-epimerase of mutant 3 (SEQ ID NO: 8) was stable up to 90°C and exhibited 100% by a treatment for 30 minutes.

### Allulose conversion rate under acidic conditions

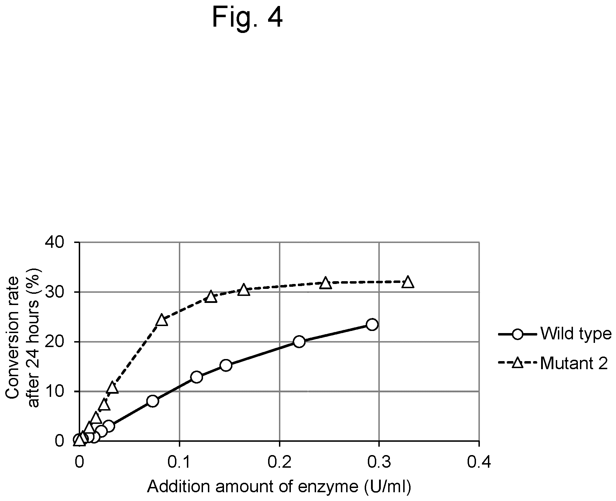

To 1 mL of a substrate solution (45.0 (w/v)% fructose, 100 mM acetate buffer, 1 mM cobalt chloride, pH 5.3), an enzyme sample (0.01 mL) diluted in an appropriate concentration was added. The mixture was treated at 75°C for 24 hours and boiled for 10 minutes to terminate the reaction. After 4 mL of water was added, fructose and allulose were detected by HPLC (column: MCI GEL CK08EC, Mitsubishi Chemical Corporation) and the allulose conversion rate was calculated based on the ratio of the peak area of fructose and the peak area of allulose (calculation formula: allulose peak area/fructose peak area × 100 (%)). The results are shown in Figure 4. In the D-allulose-3-epimerase of mutant 2 (SEQ ID NO: 6), the conversion rate after 24 hours was significantly high compared to that of wild-type (SEQ ID NO: 1) at any one of the enzyme addition amounts.

### Industrial Applicability

It is possible to produce ketose, particularly, rare sugar allulose by the ketose-3-epimerase of the present invention under a high-temperature condition.

### Sequence Listing Free Text

SEQ ID NO: 1: Wild-type sequence
SEQ ID NO: 2: Wild-type sequence (codon optimized)
SEQ ID NO: 3: A56E mutation
SEQ ID NO: 4: N57H mutation
SEQ ID NO: 5: R58G mutation
SEQ ID NO: 6: F111W mutation
SEQ ID NO: 7: A56E mutation, N57H mutation, R58G mutation
SEQ ID NO: 8: A56E mutation, N57H mutation, R58G mutation, F111W mutation
SEQ ID NO: 9: A56E mutation (codon optimized)
SEQ ID NO: 10: N57H mutation (codon optimized)
SEQ ID NO: 11: R58G mutation (codon optimized)
SEQ ID NO: 12: F111W mutation (codon optimized)
SEQ ID NO: 13: A56E mutation, N57H mutation, R58G mutation (codon optimized)
SEQ ID NO: 14: A56E mutation, N57H mutation, R58G mutation, F111W mutation (codon optimized)
SEQ ID NO: 16 to 19: primers

All publications, patents and patent applications cited herein are incorporated herein by reference in their entirety.

## Claims

1. A polypeptide shown in any one of the following (1) to (3):
(1) a polypeptide consisting of an amino acid sequence having one or two or more amino acid substitutions selected from the group consisting of A56E, N57H, R58G and F111W in the amino acid sequence shown in SEQ ID NO: 1;
(2) a polypeptide consisting of the amino acid sequence having substitution, addition, insertion or deletion of one or several amino acid residues in the amino acid sequence described in the above (1), having D-allulose-3-epimerase activity and retaining one or two or more amino acid substitutions selected from the group consisting of A56E, N57H, R58G, and F111W; and
(3) a polypeptide having a 90% or more sequence identity to the amino acid sequence described in the above (1), having D-allulose-3-epimerase activity and retaining one or two or more amino acid substitutions selected from the group consisting of A56E, N57H, R58G, and F111W.

2. The polypeptide according to claim 1, consisting of the amino acid sequence having a single substitution of F111W alone, a triple substitution of A56E, N57H, and R58G or a quadruple substitution of A56E, N57H, R58G and F111W.

3. The polypeptide according to claim 1 or 2, having improved heat resistance compared to wild-type D-allulose-3-epimerase consisting of the amino acid sequence represented by SEQ ID NO: 1, wherein the polypeptide has 60% or more residual D-allulose-3-epimerase activity when treated at 90°C for 30 minutes.

4. DNA shown in any one of the following (1) to (3):
(1) DNA encoding the polypeptide according to claim 1;
(2) DNA consisting of a nucleotide sequence represented by any one of SEQ ID NOs: 9 to 14; and
(3) DNA consisting of an equivalent sequence to the nucleotide sequence represented by any one of SEQ ID NOs: 9 to 14 and encoding a polypeptide having D-allulose-3-epimerase activity.

5. An expression cassette or recombinant vector comprising the DNA according to claim 4.

6. A transformant obtained by transforming a host with the expression cassette or recombinant vector according to claim 5.

7. A method for producing the polypeptide according to claim 1, comprising a step of culturing the transformant according to claim 6.

8. A method for producing allulose, comprising a step of allowing the polypeptide according to claim 1 to act on fructose.

9. Allulose obtained by the production method according to claim 8.

10. An enzyme agent comprising the polypeptide according to claim 1.
